# EUROPÄISCHE PATENTANMELDUNG

(11) **EP 1 197 179 A2**
(43) Veröffentlichungstag der Anmeldung: **17.04.2002**
(21) Anmeldenummer: 01122722.0
(22) Anmeldetag: 21.09.2001
(51) Int. Cl.: A61B 5/05

(54) **Verfahren und Anzeigevorrichtung zur Darstellung eines durch mehrere Grössen definierten physiologischen Zustands eines Lebewesens**

(30) Priorität: 14.10.2000 DE 10051086
(71) Anmelder: Soehnle-Waagen GmbH & Co. KG, 71540 Murrhardt (DE)
(72) Erfinder: Nahm, Werner Klaus, Dipl.-Chem., 74426 Bühlerzell (DE)

(57) **Zusammenfassung**

Die Erfindung betrifft ein Verfahren und eine Anzeigevorrichtung (1) zur Darstellung eines durch mehrere Größen definierten physiologischen Zustands (91, 92, ..., 99) eines Lebewesens. Um eine eindeutige, leicht verständliche und schnell erfassbare Darstellung des physiologischen Zustands (91, 92, ..., 99) zu ermöglichen, wird vorgeschlagen, dass die Größen jeweils einer Achse (3, 4) eines mehrdimensionalen kartesischen Koordinatensystems zugeordnet werden, dass eine Zeitbasis zur Darstellung des Zustands (91, 92, ..., 99) des Lebewesens vorgegeben wird und dass der Zustand (91, 92, ..., 99) des Lebewesens auf Grundlage der Zeitbasis in Abhängigkeit von den Werten der Größen in dem Koordinatensystem dargestellt wird. Es wird weiter vorgeschlagen, den Ursprung (8) des Koordinatensystems in den Idealzustand des Lebewesens zu legen.

## Beschreibung

Die vorliegende Erfindung betrifft ein Verfahren zur Darstellung eines durch mehrere Größen definierten. physiologischen Zustands eines Lebewesens. Die Erfindung betrifft außerdem eine Anzeigevorrichtung zur Darstellung eines durch mehrere Größen definierten Zustands eines Lebewesens, wobei die Anzeigevorrichtung einen Bildschirm zur Darstellung des Zustands aufweist.

Die vorliegende Erfindung betrifft des weiteren ein Speicherelement, insbesondere Random-Access-Memory, Read-Only-Memory oder Flash-Memory, für ein Steuergerät einer Anzeigevorrichtung zur Darstellung eines durch mehrere Größen definierten Zustands eines Lebewesens. Auf dem Speicherelement ist ein Programm abgespeichert, das auf einem Rechengerät, insbesondere auf einem Mikroprozessor, ablauffähig ist. Schließlich betrifft die Erfindung auch ein Computerprogramm.

Aus dem Stand der Technik sind Objekte allgemein bekannt, deren Zustand durch mehrere Größen definiert ist. So wird bspw. in Bronstein, Ilja Nikolaevic: "Taschenbuch der Mathematik", 23. Auflage, 1987, S. 401, Kap. 3.2.2. "Optimale Prozesse" ein geradliniger Bewegungsablauf eines Objekts betrachtet. Der Zustand des bewegten Objekts ist definiert durch die von der Zeit abhängende Ortsfunktion und Geschwindigkeit.

Aus der DE 22 46 463 ist ein als Speisepumpe ausgebildetes Objekt bekannt, dessen Zustand, der als Betriebspunkt bezeichnet wird, durch die beiden Größen Fördermenge und Druck definiert ist. Der Zustand der Speisepumpe wird in einem zweidimensionalen kartesischen Koordinatensystem dargestellt, wobei jeder Achse des Koordinatensystems eine der Größen zugeordnet ist. In dem Koordinatensystem ist ein zulässiger Betriebsbereich der Speisepumpe definiert und dargestellt. Durch die beschrieben Darstellung des Zustands der Speisepumpe kann unmittelbar abgelesen werden, ob sich der aktuelle Betriebspunkt in dem zulässigen Betriebsbereich befindet.

Aus dem Bereich der Medizin ist es bekannt, dass verschiedene physiologische Zustände eines Lebewesens durch mehrere Größen definiert sind. So ist bspw. die Fitness einer Person u. a. definiert durch die Größen Körpergewicht und Körperfettanteil. Alternativ oder zusätzlich kann die Fitness auch definiert sein durch eine oder mehrere der Größen Körperwasseranteil, Muskelmassenanteil, Fluidbalance, die als das Verhältnis der Flüssigkeitsmenge außerhalb der Körperzellen zu der Flüssigkeitsmenge innerhalb der Körperzellen definiert ist, und Bodymass-Index (BMI), der als das Verhältnis von Körpergewicht zu dem Quadrat der Körpergröße definiert ist. Das Herzinfarktrisiko einer Person ist u. a. definiert durch die Größen Blutdruck und Pulsfrequenz. Alternativ oder zusätzlich kann das Herzinfarktrisiko auch definiert sein durch eine oder mehrere der Größen Blutfett, Blutzucker und Cholesterin. Ein Wohlfühlzustand (Wellnessfaktor) einer Person ist u. a. definiert durch die Größen Umgebungstemperatur und Luftfeuchtigkeit. Alternativ oder zusätzlich kann der Wohlfühlzustand auch definiert sein durch eine oder mehrere Größen Luftdruck, Lichtstärke, Lichtverteilung oder Schalllautstärke in der Umgebung.

Zur Darstellung des physiologischen Zustands eines Lebewesens wird nach dem Stand der Technik jede den Zustand definierende Größe gesondert dargestellt. So ist es bspw. aus der EP 0 926 488 A2 bekannt, den aktuellen Zahlenwert des Körperfettanteils einer Person darzustellen. Um den zeitlichen Verlauf des Körperfettanteils über einen längeren Zeitraum hinweg verfolgen zu können, muss die Person die aktuellen Werte notieren. Aus den notierten Zahlenwerten muss dann der zeitliche Verlauf ermittelt werden. Um bspw. Informationen über die eigene Fitness zu erhalten, muss die Person zusätzlich noch ihr Körpergewicht messen, die aktuellen Zahlenwerte ebenfalls notieren und dann den zeitlichen Verlauf des Körpergewichts ermitteln. Schließlich muss die Person die notierten Größen Körperfettanteil und Körpergewicht zueinander in Relation bringen und u. U. andere Größen wie Alter und Körpergröße mit einrechnen, um eine Aussage über ihre Fitness zu erhalten. Dieses bekannte Vorgehen zur Darstellung des Zustands eines Lebewesens ist äußerst aufwendig und kompliziert. Es besteht die Gefahr, dass falsche Größen miteinander in Relation gesetzt werden, oder dass ganz einfach Rechenfehler bei der Ermittlung des Zustands auftreten.

Aus dem Stand der Technik sind sog. Körperfett-Messwaagen bekannt, die sowohl das Körpergewicht als auch den Körperfettanteil einer Person messen und darstellen. Körperfett-Messwaagen mit Speichermöglichkeit können die gemessenen Werte sogar speichern und bei Bedarf ausgeben. Dadurch erspart sich die Person das Notieren der Zahlenwerte für Körpergewicht und Körperfettanteil. Die bekannten Körperfett-Messwaagen verfügen jedoch über keinerlei Möglichkeiten, die einzelnen Größen miteinander in Relation zu setzen oder miteinander zu verknüpfen.

Die Ausgabe von gespeicherten Werten bestimmter, einen physiologischen Zustand definierenden Größen über die Zeit ist bspw. aus der DE 41 39 241 A1 bekannt. Um eine Aussage über einen durch mehrere Größen definierten Zustand machen zu können, muss die über die Zeit dargestellten Größe erst mit weiteren Größen verknüpft werden, was aufwendig und kompliziert ist.

Aus der DE 197 10 955 A1 ist es bekannt, die Fitness einer Person über der Zeit darzustellen. Bei einer solchen Darstellung eines physiologischen Zustands eines Lebewesens kann jedoch nicht nachvollzogen werden, welche Größen bei der Ermittlung des Zustands betrachtet wurden und welche aktuellen Werte die betrachteten Größen haben. So ist es bspw. möglich, dass die Fitness einer Person unzureichend ist, wenn sie einen normalen Körperfettanteil hat, gleichzeitig aber untergewichtig ist. Andererseits kann auch ein normales Körpergewicht und gleichzeitig ein erhöhter Körperfettanteil zu einer unzureichenden Fitness führen. Bei der bekannten Anzeige weiß die Person nicht, wie sie auf die angegebene unzureichende Fitness reagieren soll.

In der Praxis hat es sich gezeigt, dass die Darstellung des zeitlichen Verlaufs von einen physiologischen Zustand eines Lebewesens definierenden Größen alleine, bspw. des Körpergewichts alleine oder des Körperfettanteils alleine, nicht sehr aussagekräftig sind. Insbesondere erlaubt eine derartige Darstellung der Größen keine Aussage über den physiologischen Zustand des Lebewesens. Um darüber eine Aussage treffen zu können, müssen die einzelnen Größen erst in eine Relation zueinander gesetzt bzw. miteinander verknüpft werden. Das ist sehr aufwendig und kompliziert.

Es hat sich weiter gezeigt, dass die Akzeptanz von Vorrichtungen zur Ausgabe eines physiologischen Zustands eines Lebewesens, bspw. der Fitness, des Herzinfarktrisikos oder eines Wohlfühlzustands einer Person, ganz entscheidend von der Art der Ausgabe des Zustands abhängt. Bei heutzutage bspw. im Bereich der Intensivmedizin eingesetzten Anzeigevorrichtungen, bei denen der zeitliche Verlauf von verschiedenen, den Gesundheitszustand eines Patienten characterisierende Größen dargestellt wird, müssenen die einzelnen Anzeigewerte erst in einen Bezug zueinander gesetzt werden, um eine zuverlässige Aussage über den tatsächlichen Gesundheitszustand des Patienten treffen zu können. Insbesondere in kritischen Ausnahmesituationen kann dies zu einem fehlerhaften Ablesen der Anzeigen, zu einer Fehlinterpretation der angezeigten Werte und somit zu einer falschen Aussage über den Gesundheitszustand des Patienten führen. Auch Privatpersonen haben, insbesondere während ihrer Freizeit, keine Zeit oder Lust, einzeln dargestellte Größen erst noch miteinander zu verknüpfen, um schließlich eine Aussage über einen bestimmten physiologischen Zustand machen zu können.

Die Aufgabe der vorliegenden Erfindung besteht deshalb darin, eine eindeutige und leicht verständliche Darstellung eines physiologischen Zustands eines Lebewesens zu ermöglichen, damit der Darstellung innerhalb kürzester Zeit eine größtmögliche Informationsmenge entnommen werden kann.

Zur Lösung dieser Aufgabe schlägt die Erfindung ausgehend von dem Verfahren zur Darstellung eines physiologischen Zustands der eingangs genannten Art vor, dass jeweils eine der Größen einer Achse eines mehrdimensionalen kartesischen Koordinatensystems zugeordnet, eine Zeitbasis zur Darstellung des Zustands des Lebewesens definiert und der Zustand des Lebewesens auf Grundlage der Zeitbasis in Abhängigkeit von den Werten der Größen in dem Koordinatensystem dargestellt wird.

Erfindungsgemäß wird also vorgeschlagen, nicht den zeitlichen Verlauf der Größen einzeln darzustellen, sondern mehrere Größen, durch die der darzustellende Zustand u. a. definiert ist, in einem kartesischen Koordinatensystem gemeinsam darzustellen. Dazu wird jeder Achse des Koordinatensystems eine der Größen zugeordnet. Die einzelnen Punkte in dem Koordinatensystem entsprechen somit jeweils einem bestimmten Zustand des Lebewesens. In Abhängigkeit davon, in welchem Bereich in dem Koordinatensystem sich ein Zustand befindet, kann aus der Darstellung unmittelbar abgelesen werden, welche Werte die einzelnen Größen in dem dargestellten Zustand haben. Durch die dargestellten Größen ist zwar keine vollständige Beschreibung des physiologischen Zustands möglich, die dargestellten Größen reichen jedoch aus, um eine genaue Aussage über den Zustand des Lebewesens zu treffen.

Das erfindungsgemäße Verfahren ermöglicht eine eindeutige, leicht verständliche und schnell erfassbare Darstellung des physiologischen Zustands eines Lebewesens. Neben einer besseren Darstellung ermöglicht es das Verfahren auch bestimmte Informationen bezüglich eines physiologischen Zustands eines Lebewesens überhaupt erst darstellen zu können. Das Verfahren kann zur Ausgabe eines physiologischen Zustands im privaten oder im Freizeitbereich eingesetzt werden. Um eine Aussage über einen bestimmten physiologischen Zustand machen zu können, müssen die einzelnen Größen nicht erst noch miteinander verknüpft werden. Vielmehr kann der physiologische Zustand eines Lebewesens der Darstellung unmittelbar entnommen werden. Dadurch kann die Akzeptanz von Vorrichtungen zur Ausgabe eines physiologischen Zustands eines Lebewesens, bspw. der Fitness, des Herzinfarktrisikos oder eines Wohlfühlzustands einer Person, ganz entscheidend erhöht werden. Aber auch im professionellen medizinischen Bereich kann die erfindungsgemäße eindeutig ablesbare und schnell erfassbare Darstellung des physiologischen Zustands eines Patienten Vorteile bringen und sogar Leben retten, da die Zeit zur Ermittlung des Zustands verkürzt und die Gefahr einer falschen Ermittlung des Zustands nahezu ganz ausgeschaltet wird.

Die Werte von Größen, durch die ein physiologischer Zustand eines Lebewesens definiert wird, können entweder unmittelbar vor der Darstellung des Zustands durch geeignete Messwertaufnehmer erfasst oder aber im Vorfeld der Darstellung erfasst, in einem Speicher abgelegt und zur Darstellung aus dem Speicher abgerufen werden. Die Werte der Größen werden in einer geeigneten Verknüpfungseinheit miteinander zu dem darzustellenden Zustand verknüpft. In einer Auswerteeinheit werden dann den Werten der Größen entsprechende Bildpunkte auf einem Bildschirm ermittelt und eine Bildschirmsteuerung derart angesteuert, dass der Zustand auf dem Bildschirm dargestellt wird. Um bei mehreren dargestellten Zuständen einen Zustandsverlauf auswerten zu können, werden die dargestellten Zustände bzw. die diesen zugeordneten Werte der Größen oder die den Werten zugeordneten Bildpunkte auf dem Bildschirm in dem Speicher abgelegt. Der gesamte Ablauf der Darstellung des Zustands wird von einem Steuergerät gesteuert. Das Auswerten des Zustandsverlaufs umfasst bspw. eine Beurteilung der Güte oder Qualität des Verlaufs anhand von an sich aus der Systemtheorie bekannten Methoden, z.B. anhand von Komplexitätsmaßen oder Dimensionsmaßen.

Die Zeitbasis kann beliebig gewählt werden. Bei sich langsam ändernden Zuständen, wie der Fitness einer Person, kann sie im Bereich von Tagen gewählt werden. Bei sich schneller ändernden Zuständen, wie dem Herzinfarktrisiko eines Patienten, kann sie im Bereich von Stunden gewählt werden. Bei der Überwachung des Gesundheitszustands eines Patienten auf einer Intensivstation kann die Zeitbasis auch im Bereich von Minuten oder Sekunden oder gar noch kürzer gewählt werden.

Durch die Darstellung des Zustands eines Lebewesens auf Grundlage der Zeitbasis haben die dargestellten Zustände einen festen zeitlichen Bezug zueinander. Das bedeutet jedoch nicht, dass die Zustände in der Zeitbasis entsprechenden regelmäßigen zeitlichen Abständen dargestellt werden müssen. Vielmehr ist jedem dargestellten Zustand gewissermaßen ein Datums- und Zeitstempel zugeordnet. Eine solche zeitliche Zuordnung der Zustände kann unter bestimmten Bedingungen bei der Auswertung des Zustandsverlaufs von Bedeutung sein.

Das erfindungsgemäße Verfahren kann auch auf einem Internetserver ausgeführt werden. Dazu werden dem Server die Größen, die den physiologischen Zustand eines Lebewesens definieren, bspw. über das Internet, übermittelt. Die Größen können bspw. von einem lokalen Arbeitsplatzrechner aus eingegeben werden. Alternativ können auch geeignete Messgeräte zur Aufnahme der Größen über das Internet mit dem Server verbunden sein. Die Größen können dem Server dann unmittelbar von den Messgeräten über das Internet übermittelt werden. Auf dem Server erfolgt dann die Verarbeitung der Größen und die Berechnung von Koordinaten von darzustellenden Zustandspunkten. Die Punkte können dann bspw. auf einem Bildschirm des Arbeitsplatzrechners dargestellt werden. Dazu werden die Koordinaten der Punkte in einem geeigneten Format an den Arbeitsplatzrechner übermittelt.

Gemäß einer vorteilhaften Weiterbildung der vorliegenden Erfindung wird vorgeschlagen, dass der Ursprung des Koordinatensystems in den Idealzustand oder einen angestrebten Zielzustand des Lebewesens gelegt wird. Der angestrebte. Zielzustand kann frei gewählt werden. Der Idealzustand kann von Lebewesen zu Lebewesen variieren und wird vor der Darstellung des physiologischen Zustands ermittelt. Die Ermittlung der Werte des Idealzustands kann entweder über geeignete Modelle und/oder empirisch erfolgen. Die Werte für das Körpergewicht und den Körperfettanteil für eine ideale Fitness einer Person können bspw. anhand der Körpergröße, des Alters und des Geschlechts der Person bestimmt werden. Die Werte für den Blutdruck und die Pulsfrequenz einer Person für ein ideales bzw. minimales Herzinfarktrisiko können bspw. anhand des Alters und des Geschlechts der Person bestimmt werden. Ein idealer Wohlfühlzustand einer Person wird dagegen eher empirisch ermittelt werden, indem die Person einer bestimmten Umgebungstemperatur in Verbindung mit einer bestimmten Luftfeuchtigkeit ausgesetzt und ihre Reaktion bzw. Beurteilung bewertet wird. Der ideale Wohlfühlzustand kann bspw. in Abhängigkeit von dem Geschlecht, dem Alter, dem Gesundheitszustand der Person oder anderen Faktoren variieren.

Dadurch dass der Ursprung des Koordinatensystems in den Idealzustand des Lebewesens gelegt wird, kann auf einen Blick erfasst werden, wie weit der aktuelle Zustand von dem Idealzustand entfernt ist und welche Größen wie beeinflusst werden müssen, damit der Idealzustand erreicht wird. Dieser Aspekt ist bspw. bei der Abarbeitung von Trainingsprogrammen von Bedeutung, da der erfindungsgemäßen Darstellung der Fitness unmittelbar entnommen werden kann, inwieweit das Körpergewicht oder der Körperfettanteil verringert werden müssen, um die ideale Fitness zu erreichen. Ebenso kann der Darstellung eines bestimmten physiologischen Zustands eines Patienten im professionellen medizinischen Bereich unmittelbar entnommen werden, ob der Zustand ideal ist oder ob und inwieweit auf eine Veränderung bestimmter Größen, durch die der dargestellte Zustand definiert ist, hingewirkt werden muss, um den Idealzustand zu erreichen.

Gemäß einer bevorzugten Ausführungsform der vorliegenden Erfindung wird vorgeschlagen, dass ein idealer Verlauf des Zustands des Lebewesens ausgehend von einem beliebigen Zustand bis in den Ursprung des Koordinatensystems ermittelt wird. Es ist denkbar, dass der Idealverlauf ausgehend von dem ersten dargestellten Zustand bis in den Ursprung des Koordinatensystems einmal ermittelt und dann beibehalten wird. Der Idealverlauf kann aber auch bei jedem neu dargestellten Zustand neu ermittelt werden. Der ideale Verlauf muss nicht unbedingt der kürzeste Weg von einem beliebigen Zustand in den Ursprung des Koordinatensystems sein. Insbesondere bei physiologischen Zuständen kann es aus gesundheitlichen Gründen nämlich erforderlich sein, auf dem Weg zu dem Idealzustand eine bestimmte Zustandsbahn abzufahren. Wenn bspw. bei dem Zustand der Fitness eine Person einen erhöhten Körperfettanteil und ein erhöhtes Körpergewicht aufweist, sollte zunächst das Körpergewicht und dann allmählich auch der Körperfettanteil reduziert werden. Ebenso sollte, wenn bspw. bei dem Zustand der Fitness eine Person einen erhöhten Körperfettanteil und ein niedriges Körpergewicht aufweist, die Person zur Verringerung des Körperfettanteils nicht einfach weniger Essen. Dadurch würde nämlich das Körpergewicht weiter verringert, was zu Untergewicht führen kann. Die Person sollte vielmehr gezielten Muskelaufbau betreiben. Dadurch würde nämlich sowohl das Körpergewicht aufgrund der zusätzlichen Muskelmasse erhöht als auch der Körperfettanteil (als auf das Körpergewicht bezogene Größe) verringert.

Es ist denkbar, dass ermittelt wird, wie auf das Lebewesen eingewirkt werden muss, damit der Zustand des Lebewesens dem idealen Verlauf folgt. Bspw. bei dem Zustand der Fitness kann der Körperfettanteil durch einen Aufbau von Muskeln und das Körpergewicht durch eine Diät vermindert werden. Durch einen bestimmten Muskelaufbau und eine bestimmte Diät kann die Fitness einer Person gezielt beeinflusst werden, vorzugsweise derart, dass sie dem idealen Verlauf folgt. Es wird also ein idealer Diät- und Trainingsplan erstellt, der von einer Person abgearbeitet werden sollte, damit ihre Fitness dem idealen Zustandsverlauf bis zu dem Idealzustand im Ursprung des Koordinatensystems folgt. Im professionellen medizinischen Bereich kann bspw. ermittelt werden, welche Medikamente einem Patienten verabreicht werden müssen, damit dessen Gesundheitszustand einem Idealverlauf folgt.

Vorteilhafterweise wird der ideale Verlauf des Zustands des Lebewesens ausgehend von einem ersten dargestellten Zustand bis in den Ursprung des Koordinatensystems in dem Koordinatensystem dargestellt. Der dargestellte Idealverlauf dient gewissermaßen als Soll-Verlauf des physiologischen Zustands des Lebewesens. Aus einem Vergleich des Soll-Verlaufs mit dem tatsächlichen Ist-Verlauf des Zustands kann bspw. der Erfolg von bestimmten Maßnahmen überprüft werden, die ausgeführt wurden, um das Lebewesen in den Idealzustand zu überführen.

Um das Ablesen des dargestellten Zustands weiter zu erleichtern, wird gemäß einer weiteren bevorzugten Ausführungsform der vorliegenden Erfindung vorgeschlagen, dass in dem Koordinatensystem verschiedene Gütebereiche definiert und dargestellt werden, in denen der Zustand des Lebewesens unterschiedlich gut ist. Die verschiedenen Gütebereiche können bspw. durch verschiedene Farben oder durch verschiedene Muster gekennzeichnet werden. Im einfachsten Fall erstrecken sich die Gütebereiche vom Ursprung des Koordinatensystems aus kreisförmig nach außen. Je weiter außen ein Gütebereich liegt, desto schlechter ist der Zustand des Lebewesens. In anderen Fällen ist es bspw. denkbar, dass Bereiche gleicher Güte in dem Koordinatensystem unsymmetrisch ausgebildet sind. So ist bspw. bei dem Zustand der Fitness ein hoher Körperfettanteil unabhängig von dem Körpergewicht schlecht. Ein geringer Körperfettanteil ist in Verbindung mit einem Untergewicht schlecht, in Verbindung mit einem Normal- oder Übergewicht jedoch gut.

Gemäß einer anderen vorteilhaften Weiterbildung der vorliegenden Erfindung wird vorgeschlagen, dass der physiologische Zustand des Lebewesens in einem zweidimensionalen Koordinatensystem dargestellt wird. Der physiologische Zustand des Lebewesens wird zwar durch mehrere Größen definiert, dargestellt werden jedoch nur zwei ausgewählte der Größen. Dies kann bspw. durch Projektion der mehrdimensionalen Darstellung oder durch einen Schnitt durch die mehrdimensionale Darstellung des Zustands erfolgen. Vorteilhafterweise wird der physiologische Zustand des Lebewesens durch mindestens zwei der nachfolgend aufgeführten Größen Körpergewicht, Körperfettanteil, Körperwasseranteil, Muskelmassenanteil, Fluidbalance, die als das Verhältnis der Flüssigkeitsmenge außerhalb der Körperzellen zu der Flüssigkeitsmenge innerhalb der Körperzellen definiert ist, Bodymass-Index (BMI), der als das Verhältnis von Körpergewicht zu dem Quadrat der Körpergröße definiert ist, Blutdruck, Pulsfrequenz, Blutfett, Blutzucker oder Cholesterin des Lebewesens definiert. Anhand dieser Größen können Fitness, Herzinfarktrisiko und andere physiologischen Zustände definiert werden.

Alternativ wird der physiologische Zustand des Lebewesens durch mindestens zwei der nachfolgend aufgeführten Größen Luftdruck, Umgebungstemperatur, Luftfeuchtigkeit, Lichtstärke, Lichtverteilung oder Schalllautstärke in der Umgebung definiert. Anhand dieser Größen kann bspw. ein Wohlfühlzustand definiert werden.

Für bestimmte Anwendungen wäre es interessant, der Darstellung nicht nur den Zustandsverlauf, sondern auch die Geschwindigkeit einer Zustandsänderung entnehmen zu können. Der aus dem Stand der Technik bekannten Darstellung des zeitlichen Verlaufs kann die Geschwindigkeit einer Zustandsänderung dem Gradienten des Verlaufs entnommen werden. Der erfindungsgemäßen Darstellung kann die Geschwindigkeit einer Zustandsänderung nur in dem Sonderfall ohne weiteres entnommen werden, dass die einen Zustand definierenden Größen in äquidistanten zeitlichen Abständen vorliegen, d. h. erfasst werden oder abgespeichert sind. Wenn die Zustände dann in dem Koordinatensystem dargestellt werden, ergibt sich die Geschwindigkeit der Zustandsänderung aus den Abständen zwischen zwei aufeinanderfolgenden Zuständen. Ein großer Abstand bedeutet eine schnelle Zustandsänderung, ein kleiner Abstand eine langsame Zustandsänderung. Vorteilhafterweise liegen deshalb die Werte jeder Größe in der Zeitbasis entsprechenden äquidistanten zeitlichen Abständen vor.

Wenn die einen Zustand definierenden Größen jedoch in beliebigen zeitlichen Abständen vorliegen, muss eine andere Möglichkeit geschaffen werden, der Darstellung die Geschwindigkeit der Zustandsänderung entnehmen zu können. Zu diesem Zweck wird gemäß einer weiteren bevorzugten Ausführungsform der vorliegenden Erfindung vorgeschlagen, dass die Geschwindigkeit der Änderung eines Zustands des Lebewesens durch eine auf die Zeitbasis bezogene Codierung der dargestellten Zustände in dem Koordinatensystem dargestellt wird. Vorteilhafterweise wird die auf die Zeitbasis bezogene Codierung über unterschiedliche Strichstärken, unterschiedliche Farben oder unterschiedlichen Kontrast der dargestellten Zustände oder von Verbindungslinien zwischen den dargestellten Zuständen erzielt.

Gemäß noch einer weiteren bevorzugten Ausführungsform der vorliegenden Erfindung wird vorgeschlagen, dass die Werte jeder Größe in beliebigen zeitlichen Abständen vorliegen und bei mehreren Werten einer Größe pro Zeitbasis die Werte der Größe zu einem gemeinsamen Wert der Größe zusammengefaßt werden und der Zustand des Lebewesens auf Grundlage der Zeitbasis in Abhängigkeit von dem gemeinsamen Wert der Größe in dem Koordinatensystem dargestellt wird. Aus den mehreren Werten einer Größe kann durch Mittelwertbildung, Summenbildung oder auf eine beliebige andere Weise ein gemeinsamer Wert der Größe gebildet werden. Auch die Gewichtung der einzelnen Werte bzw. der einzelnen Zustände mit verschiedenen Gewichtungsfaktoren ist denkbar. Diese Ausführungsform hat den Vorteil, dass - wie bei den zeitlich äquidistant vorliegenden Werten - die Abstände aufeinanderfolgend dargestellter Zustände ein Maß für die Geschwindigkeit der Änderung des Zustands sind.

Alternativ wird vorgeschlagen, dass die Werte jeder Größe in beliebigen zeitlichen Abständen vorliegen und bei weniger als einem Wert einer Größe pro Zeitbasis fehlende Werte der Größe aus den vorhandenen Werten der Größe durch Ausgleichsrechnung bestimmt werden und der Zustand des Lebewesens auf Grundlage der Zeitbasis in Abhängigkeit von dem durch Ausgleichsrechnung bestimmten Wert der Größe in dem Koordinatensystem dargestellt wird. Zur Ausgleichsrechnung kann insbesondere eine Interpolation, eine Extrapolation oder eine Approximation eingesetzt werden (vgl. Schrüfer, Elmar: "Signalverarbeitung: numerische Verarbeitung digitaler Signale", Carl Hanser Verlag, 1990, Kapitel 3, "Ausgleichsrechnung"). Mit Hilfe der Ausgleichsrechnung können Zustände zu beliebigen Zeitpunkten aus den vor diesem Zeitpunkt ermittelten Werten der Größen modelliert werden. Auch diese Alternative hat den Vorteil, dass - wie bei den zeitlich äquidistant vorliegenden Werten - die Abstände aufeinanderfolgend dargestellter Zustände ein Maß für die Geschwindigkeit der Änderung des Zustands sind.

Gemäß noch einer anderen vorteilhaften Weiterbildung der vorliegenden Erfindung wird vorgeschlagen, dass ein Erfolgsindex ermittelt und dargestellt wird, der sich aus einem Vergleich des idealen Verlaufs mit dem tatsächlichen Verlauf des Zustands des Lebewesens ergibt. Der Erfolgsindex erlaubt auf eine einfache Weise eine Überwachung einer angestrebten Zustandsänderung. Anhand des Erfolgsindex kann bspw. der Erfolg einer Diät und/oder eines Muskelaufbautrainings auf die Fitness einer Person oder die Wirkung von bestimmten Medikamenten oder anderer Therapien auf den Gesundheitszustand eines Patienten überwacht werden. Vorteilhafterweise wird für jeden dargestellten Zustand ein Vergleich eines Verbindungsvektors zu einem nachfolgend erfassten Zustand mit einem Verbindungsvektor zu einem nachfolgenden Zustand des idealen Verlaufs ausgeführt.

Auch bei diesem Verfahren zur Darstellung eines durch mehrere Größen definierten physiologischen Zustands eines Lebewesens wird vorteilhafterweise jeweils eine der Größen einer Achse eines mehrdimensionalen kartesischen Koordinatensystems zugeordnet, eine Zeitbasis zur Darstellung des Zustands des Lebewesens definiert und der Zustand des Lebewesens auf Grundlage der Zeitbasis in Abhängigkeit von den Werten der Größen in dem Koordinatensystem dargestellt. Die Ermittlung und Ausgabe des Erfolgsindex hat die angegebenen Vorteile aber auch ohne diese Merkmale des Kennzeichnes des Patentanspruchs 1. Der Schutz des Patents soll sich deshalb auch auf ein Verfahren zur Darstellung eines physiologischen Zustands eines Lebewesens beziehen, bei dem die im Kennzeichen des Patentanspruchs 1 genannten Merkmale fehlen.

Von besonderer Bedeutung ist die Realisierung des erfindungsgemäßen Verfahrens in der Form eines Speicherelements, das für ein Steuergerät einer Anzeigevorrichtung zur Darstellung eines durch mehrere Größen definierten Zustands eines Lebewesens vorgesehen ist. Dabei ist auf dem Speicherelement ein Programm abgespeichert, das auf einem Rechengerät, insbesondere auf einem Mikroprozessor, ablauffähig und zur Ausführung des erfindungsgemäßen Verfahrens geeignet ist. In diesem Fall wird also die Erfindung durch ein auf dem Speicherelement abgespeichertes Programm realisiert, so dass dieses mit dem Programm versehene Speicherelement in gleicher Weise die Erfindung darstellt wie das Verfahren, zu dessen Ausführung das Programm geeignet ist. Als Speicherelement kann insbesondere ein elektrisches Speichermedium zur Anwendung kommen, bspw. ein Random-Access-Memory, ein Read-Only-Memory oder ein Flash-Memory.

Als eine weitere Lösung der Aufgabe der vorliegenden Erfindung wird ausgehend von der Anzeigevorrichtung zur Darstellung eines Zustands eines Lebewesens der eingangs genannten Art vorgeschlagen, dass auf dem Bildschirm ein mehrdimensionales kartesisches Koordinatensystem definiert ist, wobei jeweils einer Achse des Koordinatensystems eine der Größen zugeordnet ist, und auf dem Bildschirm der Zustand des Lebewesens in dem Koordinatensystem darstellbar ist, wobei die Darstellung auf Grundlage einer vorgebbaren Zeitbasis in Abhängigkeit von den Werten der Größen erfolgt.

Als noch eine weitere Lösung der Aufgabe der vorliegenden Erfindung wird ausgehend von dem Steuergerät einer Anzeigevorrichtung der eingangs genannten Art vorgeschlagen, dass das Steuergerät die Definition eines mehrdimensionalen kartesischen Koordinatensystems auf Mitteln der Anzeigevorrichtung veranlasst, wobei jeweils einer Achse des Koordinatensystems eine der Größen zugeordnet ist, und das Steuergerät die Darstellung des Zustands des Lebewesens in dem Koordinatensystem auf den Mitteln veranlasst, wobei die Darstellung auf Grundlage einer vorgebbaren Zeitbasis in Abhängigkeit von den Werten der Größen erfolgt.

Schließlich wird als eine Lösung der Aufgabe der vorliegenden Erfindung ein Computerprogramm vorgeschlagen, das zur Durchführung des erfindungsgemäßen Verfahrens geeignet ist, wenn es auf einem Rechengerät, insbesondere auf einem Mikroprozessor, ausgeführt wird. Das Computerprogramm ist vorteilhafterweise auf einem Speicherelement, insbesondere auf einem Flash-Memory, abgespeichert.

Weitere Merkmale, Anwendungsmöglichkeiten und Vorteile der Erfindung ergeben sich aus der nachfolgenden Beschreibung von Ausführungsbeispielen der Erfindung, die in der Zeichnung dargestellt sind. Dabei bilden alle beschriebenen oder dargestellten Merkmale für sich oder in beliebiger Kombination den Gegenstand der Erfindung, unabhängig von ihrer Zusammenfassung in den Patentansprüchen oder deren Rückbeziehung sowie unabhängig von ihrer Formulierung bzw. Darstellung in der Beschreibung bzw. in der Zeichnung. Es zeigen:
- Fig. 1: eine erfindungsgemäße Anzeigevorrichtung gemäß einer ersten bevorzugten Ausführungsform der Erfindung;
- Fig. 2: eine erfindungsgemäße Anzeigevorrichtung gemäß einer zweiten bevorzugten Ausführungsform der Erfindung;
- Fig. 3: einen Ausschnitt aus der Darstellung der Anzeigevorrichtungen aus Fig. 1 und Fig. 2;
- Fig. 4: ein Ablaufdiagramm eines erfindungsgemäßen Verfahrens gemäß einer bevorzugten Ausführungsform der Erfindung; und
- Fig. 5: ein erfindungsgemäßes Steuergerät gemäß einer bevorzugten Ausführungsform.

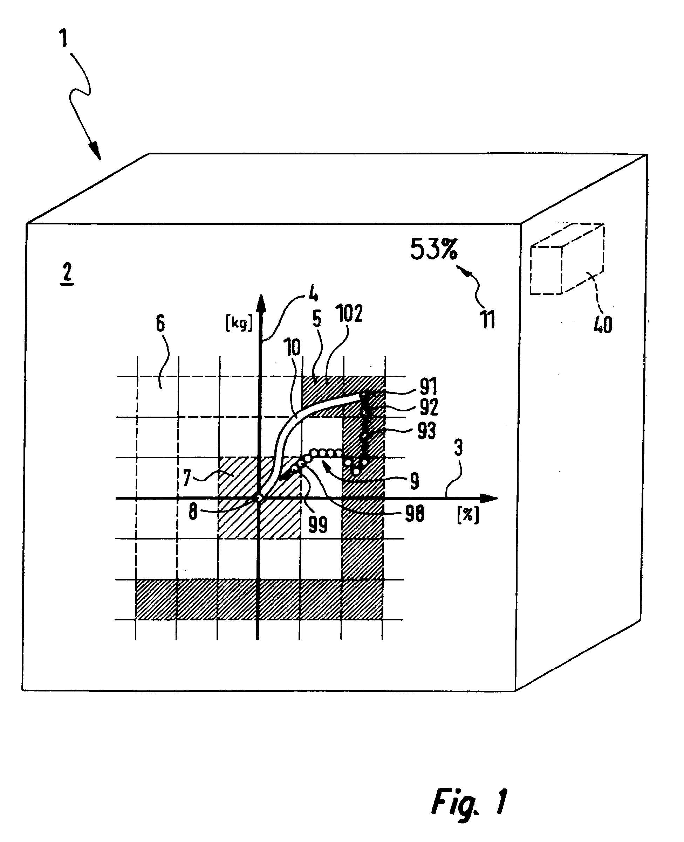

In Fig. 1 ist eine erfindungsgemäße Anzeigevorrichtung in ihrer Gesamtheit mit dem Bezugszeichen 1 bezeichnet. Die Anzeigevorrichtung 1 weist einen Bildschirm 2 auf, der bspw. als ein Röhrenbildschirm, ein LCD-, TFT- oder beliebig anderes Display ausgebildet ist. Auf dem Bildschirm 2 ist ein zweidimensionales kartesisches Koordinatensystem mit einer X-Achse 3 und einer Y-Achse 4 und ein durch zwei Größen definierter physiologischer Zustand eines Lebewesens dargestellt.

In dem vorliegenden Ausführungsbeispiel ist als Zustand die Fitness einer Person dargestellt. Die Fitness ist u. a. definiert durch die Größen Körpergewicht g und Körperfettanteil f. Der X-Achse 3 ist der Körperfettanteil f und der Y-Achse 4 das Körpergewicht g zugeordnet. Der Körperfettanteil f ist einheitslos und wird in Prozent (%) angegeben. Das Körpergewicht g hat die Einheit Kilogramm (kg).

Jeder Zustand ist somit durch ein Wertepaar aus einem Wert des Körperfettanteils f und einem Wert des Körpergewichts g charakterisiert. Jeder Zustand wird durch einen Punkt in dem Koordinatensystem repräsentiert. Wenn mehrere aufeinanderfolgende Zustände dargestellt sind, ergibt sich ein bestimmter Zustandsverlauf. Darüber hinaus ist jedem Zustand ein Datums- und/oder Zeitstempel zugeordnet, der eine zeitliche Zuordnung der einzelnen Zustände und damit eine zeitliche Auswertung des Zustandsverlaufs erlaubt. Als zeitliche Auswertung ist insbesondere an die Ausgabe der Geschwindigkeit von Änderungen des Zustandsverlaufs gedacht.

Die erfindungsgemäße Darstellung ermöglicht eine eindeutige, leicht verständliche und schnell erfassbare Darstellung des physiologischen Zustands eines Lebewesens. Die dargestellten Zustände beruhen entweder auf aktuell erfassten Werten der Größen Körpergewicht g und Körperfettanteil f oder auf früher erfassten und dann abgespeicherten Werten der Größen.

Der Ursprung 8 des Koordinatensystems ist in den Idealzustand oder in einen beliebigen angestrebten Zielzustand des Lebewesens gelegt. Der Idealzustand eines Lebewesens kann entweder durch ein geeignetes Modell und/oder empirisch ermittelt werden. Die Idealwerte für das Körpergewicht und den Körperfettanteil für eine ideale Fitness einer Person können bspw. anhand der Körpergröße, des Alters und des Geschlechts der Person bestimmt werden.

In dem Koordinatensystem auf dem Bildschirm 2 sind auch drei verschiedene Gütebereiche 5, 6, 7 dargestellt, in denen der Zustand des Lebewesens unterschiedlich gut ist. Die verschiedenen Gütebereiche 5, 6, 7 sind durch unterschiedliche Muster gekennzeichnet. Es wäre jedoch auch eine Kennzeichnung durch unterschiedliche Graustufen denkbar. Ebenso wäre bei einer farbigen Darstellung des Zustands auch eine Kennzeichnung der Gütebereiche 5, 6, 7 durch unterschiedliche Farben denkbar. Der Gütebereich 5, wo der Zustand des Lebewesens am schlechtesten ist, ist dicht schraffiert dargestellt. Der Gütebereich 7, wo der Zustand des Lebewesens am besten ist, ist leicht schraffiert und der mittlere Gütebereich 6 ist ohne Schraffur dargestellt. Die einzelnen Gütebereiche 5, 6, 7 können beliebig oft und beliebig fein unterteilt werden, um den Zustand des Lebewesens auf einen Blick beliebig genau bestimmten zu können.

Der Verlauf des Zustands ist in Fig. 1 mit dem Bezugszeichen 9 bezeichnet. Die einzelnen Zustände sind mit 91, 92, ..., 99 bezeichnet. Auf dem Bildschirm 2 ist des weiteren ein idealer Verlauf 10 des Zustands des Lebewesens ausgehend von dem ersten dargestellten Zustand 91 bis in den Ursprung 8 des Koordinatensystems dargestellt. Schließlich ist auf dem Bildschirm 2 auch ein Erfolgsindex 11 dargestellt, der aus einem Vergleich des idealen Verlaufs 10 mit dem tatsächlichen Verlauf 9 des Zustands des Lebewesens ermittelt wird. Dabei wird ausgehend von jedem dargestellten Zustand 91, 92, ..., 99 ein idealer Verlauf 10 zu dem Ursprung 8 ermittelt.

Der Darstellung des Verlaufs 9 des physiologischen Zustands 91, 92, ..., 99 des Lebewesens auf dem Bildschirm 2 können Informationen entnommen werden, die einer herkömmlichen Anzeige eines zeitlichen Verlaufs einer oder mehrerer Größen nicht entnommen werden können. Wenn bspw. eine Person bei dem Zustand der Fitness einen erhöhten Körperfettanteil f aufweist, wird sie bei einer herkömmlichen Anzeige als erste Reaktion weniger bzw. magerer essen, um den Körperfettanteil f zu reduzieren. Wenn der Körperfettanteil f jedoch nach dem erfindungsgemäßen Verfahren zusammen mit dem Körpergewicht g dargestellt wird, kann der Darstellung bei einem niedrigen Körpergewicht g der Person entnommen werden, dass weniger bzw. magerer zu essen lediglich zu einer weiteren Verringerung des Körpergewichts g und schließlich zu einem weiteren Anstieg des Körperfettanteils f führt, da der Körperfettanteil f auf das Körpergewicht g bezogen ist. Der erfindungsgemäßen Darstellung kann dagegen entnommen werden, dass die Person stattdessen vielmehr gezielten Muskelaufbau betreiben sollte, was zu einer Erhöhung des Körpergewichts g aufgrund der zusätzlichen Muskelmasse und damit zu einer Verringerung des Körperfettanteils f führt.

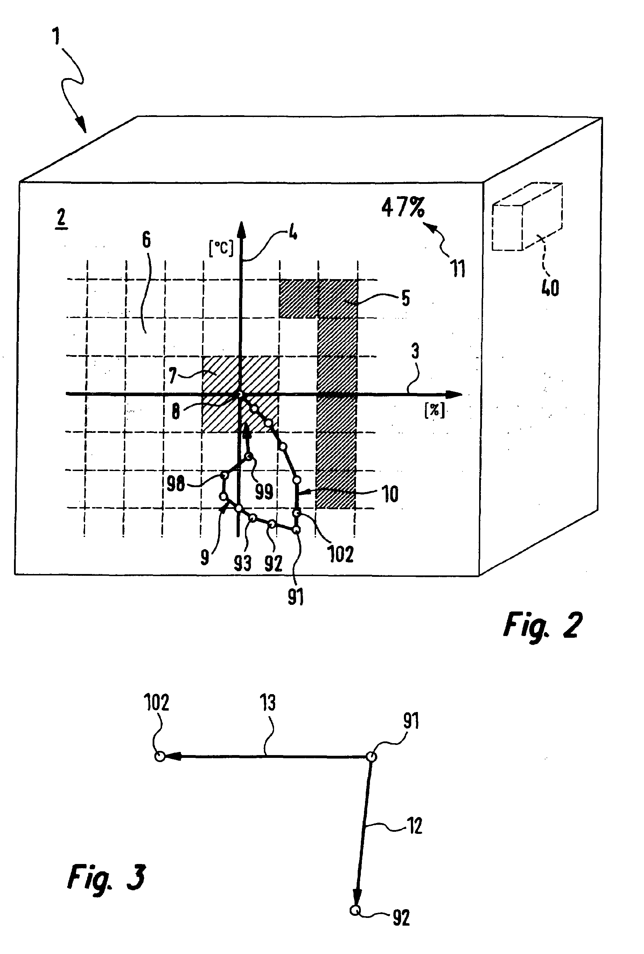

In Fig. 3 ist die Ermittlung des Erfolgsindex 11 beispielhaft für den Zustand 91 dargestellt. Für den dargestellten Zustand 91 wird ein Verbindungsvektor 12 zu dem nachfolgend erfassten Zustand 92 mit einem Verbindungsvektor 13 zu einem nachfolgenden Zustand 102 des idealen Verlaufs 10 verglichen. Bei deckungsgleichen Verbindungsvektoren 12, 13 kann der Erfolgsindex 11 bspw. als 1.0 oder 100% definiert werden. Bei genau entgegengesetzt zueinander verlaufenden Verbindungsvektoren 12, 13 ist der Erfolgsindex 11 bspw. als 0.0 oder 0% definiert. In dem Ausführungsbeispiel aus Fig. 3 beträgt der Erfolgsindex 11 etwa 53%. In dem Ausführungsbeispiel aus Fig. 1 ist der Erfolgsindex 11 zu Beginn des Verlaufs 9 relativ schlecht und gegen Ende des Verlaufs 9 besser.

Der auf dem Bildschirm 2 dargestellte physiologische Zustand könnte statt durch Körpergewicht und Körperfett auch durch eine beliebige Kombination von zwei der nachfolgend aufgeführten Größen Körpergewicht, Körperfettanteil, Körperwasseranteil, Muskelmassenanteil, Fluidbalance, die als das Verhältnis der Flüssigkeitsmenge außerhalb der Körperzellen zu der Flüssigkeitsmenge innerhalb der Körperzellen definiert ist, Bodymass-Index (BMI), der als das Verhältnis von Körpergewicht zu dem Quadrat der Körpergröße definiert ist, Blutdruck, Pulsfrequenz, Blutfett, Blutzucker oder Cholesterin des Lebewesens definiert sein. Statt der Fitness einer Person könnte auch das Herzinfarktrisiko oder der allgemeine Gesundheitszustand einer Person als physiologischer Zustand eines Lebewesens auf dem Bildschirm 2 dargestellt werden.

In Fig. 2 ist ein weiteres Ausführungsbeispiel der vorliegenden Erfindung dargestellt, wobei dort als physiologischer Zustand ein Wohlfühl- oder Wellnessfaktor einer Person auf dem Bildschirm 2 dargestellt ist. Der Wohlfühlfaktor kann durch eine beliebige Kombination von zwei oder mehreren der nachfolgend aufgeführten Größen Luftdruck, Umgebungstemperatur, Luftfeuchtigkeit, Lichtstärke, Lichtverteilung, Schalllautstärke, Schallfrequenz, Farben und Gerüche in der Umgebung definiert sein. In dem Ausführungsbeispiel aus Fig. 2 ist der Wohlfühlfaktor definiert durch die relative Luftfeuchtigkeit in Prozent (%) (X-Achse 3) und die Umgebungstemperatur in Grad Celsius (°C) (Y-Achse 4).

Der Ursprung 8 des Koordinatensystems ist in den Idealzustand des Lebewesens gelegt. Die Idealwerte für die Luftfeuchtigkeit und die Umgebungstemperatur für einen idealen Wohlfühlfaktor einer Person können bspw. empirisch ermittelt werden, indem die Person einer bestimmten Umgebungstemperatur in Verbindung mit einer bestimmten Luftfeuchtigkeit ausgesetzt und ihre Reaktion bzw. Beurteilung bewertet wird. Der ideale Wohlfühlzustand kann bspw. in Abhängigkeit von dem Geschlecht, dem Alter, dem Gesundheitszustand der Person variieren. Deshalb wird der Idealzustand des Wohlfühlfaktors für jede Person individuell ermittelt.

Für den Wohlfühlfaktor sind Gütebereiche 5, 6, 7 ermittelt, in denen der Zustand des Lebewesens unterschiedlich gut ist, und in dem Koordinatensystem dargestellt. Der Gütebereich 5, wo der Zustand des Lebewesens am schlechtesten ist, ist dicht schraffiert dargestellt. Es ist zu erkennen, dass eine hohe Luftfeuchtigkeit unabhängig von der Umgebungstemperatur von der Person immer als unangenehm empfunden und einen schlechten Wohlfühlzustand zur Folge hat. Bei hohen Umgebungstemperaturen führt bereits eine geringere Luftfeuchtigkeit zu einem schlechten Wohlfühlzustand der Person. Der Gütebereich 7, wo der Zustand des Lebewesens am besten ist, ist leicht schraffiert und der mittlere Gütebereich 6 ist ohne Schraffur dargestellt.

Ausgehend von einem ersten dargestellten Zustand 91 ist in Fig. 2 ein Verlauf 9 der Zustände 91, 92, ..., 99 dargestellt. Ebenso ist ausgehend von dem ersten Zustand 91 ein idealer Verlauf 10 bis in den Ursprung 8 des Koordinatensystems dargestellt. Der Darstellung kann unmittelbar entnommen werden, dass zu Beginn die Luftfeuchtigkeit zu stark und die Temperatur zu wenig verringert wurde. Dadurch ergab sich eine Abweichung des tatsächlichen Verlaufs 9 von dem idealen Verlauf 10.

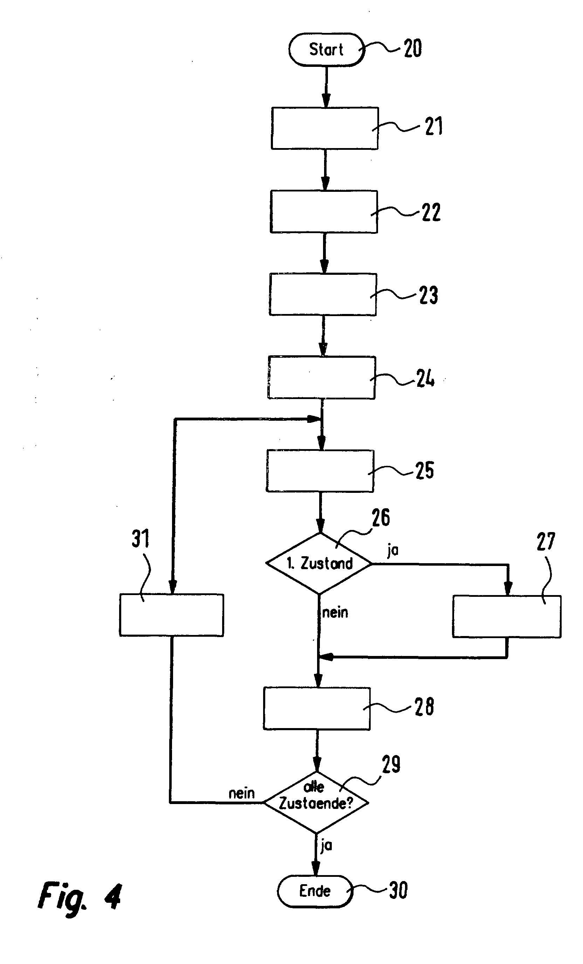

In Fig. 4 ist ein Ablaufdiagramm eines erfindungsgemäßen Verfahrens dargestellt. Das Verfahren beginnt in einem Funktionsblock 20. Anschließend wird in einem Funktionsblock 21 den Achsen 3, 4 des kartesischen Koordinatensystems jeweils eine der Größen zugeordnet, durch die der auf dem Bildschirm 2 der Anzeigevorrichtung 1 dargestellte Zustand 91, 92, ..., 99 definiert ist. In einem Funktionsblock 22 wird eine Zeitbasis zur Darstellung des Zustands 91, 92, ..., 99 vorgegeben. Anschließend wird in Funktionsblock 23 ein idealer Zustand des Lebewesens ermittelt und der Ursprung 8 des Koordinatensystems in den Ursprung 8 gelegt. Des weiteren werden in einem Funktionsblock 24 Gütebereiche 5, 6, 7 ermittelt, in denen der Zustand des Lebewesens unterschiedlich gut ist, und auf dem Bildschirm 2 dargestellt. Dann wird in einem Funktionsblock 25 ein physiologischer Zustand 91, 92, ..., 99 des Lebewesens, dargestellt.

In einem Abfrageblock 26 wird überprüft, ob der dargestellte Zustand 91, 92, ..., 99 der erste auf dem Bildschirm 2 dargestellte Zustand ist. Falls das der Fall ist, wird in einem Funktionsblock 27 ausgehend von dem ersten dargestellten Zustand 91 ein idealer Verlauf 10 bis in den Ursprung 8 des Koordinatensystems ermittelt und auf dem Bildschirm 2 dargestellt. In einem Funktionsblock 28 wird aus einem Vergleich des idealen Verlaufs 10 mit dem tatsächlichen Verlauf 9 des Zustands 91, 92, ..., 99 des Lebewesens ein Erfolgsindex 11 ermittelt und auf dem Bildschirm 2 dargestellt.

In einem Abfrageblock 29 wird überprüft, ob alle Zustände 91, 92, ..., 99 in dem Koordinatensystem dargestellt worden sind. Falls ja, wird das Verfahren in einem Funktionsblock 30 beendet. Anderenfalls wird zu einem Funktionsblock 31 verzweigt, wo der nachfolgende Zustand 92, 93, ..., 99 ausgewählt und dann in Funktionsblock 25 dargestellt wird.

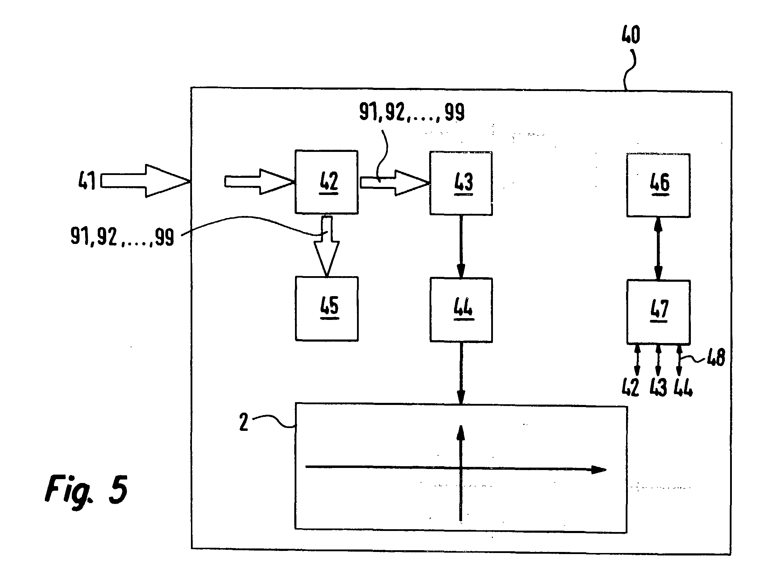

Der gesamte Ablauf der Darstellung des Zustands 91, 92, ..., 99 wird von einem Steuergerät 40 (vgl. Fig. 5) gesteuert. Das Steuergerät 40 weist ein Speicherelement 46 auf, auf dem ein Programm abgespeichert ist, das auf einem Mikroprozessor 47 des Steuergeräts 40 ablauffähig und zur Ausführung des erfindungsgemäßen Verfahrens geeignet ist. Die Werte von Größen, durch die ein physiologischer Zustand 91, 92, ..., 99 eines Lebewesens definiert wird, können entweder unmittelbar vor der Darstellung des Zustands 91, 92, ..., 99 durch geeignete Messwertaufnehmer 41 erfasst oder aber im Vorfeld der Darstellung erfasst, in einem Speicher abgelegt und zur Darstellung aus dem Speicher abgerufen werden. Die Werte der Größen werden in einer geeigneten Verknüpfungseinheit 42 miteinander zu dem darzustellenden Zustand 91, 92, ..., 99 verknüpft. In einer Auswerteeinheit 43 werden dann den Werten der Größen entsprechende Bildpunkte auf einem Bildschirm 2 ermittelt und eine Bildschirmsteuerung 44 derart angesteuert, dass der Zustand 91, 92, ..., 99 auf dem Bildschirm 2 dargestellt wird. Um bei mehreren dargestellten Zuständen 91, 92, ..., 99 einen Zustandsverlauf auswerten zu können, werden die dargestellten Zustände 91, 92, ..., 99 bzw. die diesen zugeordneten Werte der Größen oder die den Werten zugeordneten Bildpunkte in einem Speicher abgelegt. Der Mikroprozessor 47 ist über eine geeignete Datenverbindung 48 (Leitungen oder Bus) mit der Verknüpfungseinheit 42, der Auswerteeinheit 43 und der Bildschirmsteuerung 44 verbunden.

## Patentansprüche

1. Verfahren zur Darstellung eines durch mehrere Größen definierten physiologischen Zustands (91, 92, ..., 99) eines Lebewesens, **dadurch gekennzeichnet, dass** jeweils eine der Größen einer Achse (3, 4) eines mehrdimensionalen kartesischen Koordinatensystems zugeordnet, eine Zeitbasis zur Darstellung des Zustands (91, 92, ..., 99) des Lebewesens definiert und der Zustand (91, 92, ..., 99) des Lebewesens auf Grundlage der Zeitbasis in Abhängigkeit von den Werten der Größen in dem Koordinatensystem dargestellt wird.

2. Verfahren nach Anspruch 1, **dadurch gekennzeichnet, dass** der Ursprung (8) des Koordinatensystems in den Idealzustand oder einen angestrebten Zielzustand des Lebewesens gelegt wird.

3. Verfahren nach Anspruch 2, **dadurch gekennzeichnet, dass** ein idealer Verlauf (10) des Zustands des Lebewesens ausgehend von einem beliebigen Zustand (91, 92, ..., 99) bis in den Ursprung (8) des Koordinatensystems ermittelt wird.

4. Verfahren nach Anspruch 3, **dadurch gekennzeichnet, dass** der ideale Verlauf (10) des Zustands des Lebewesens ausgehend von einem ersten dargestellten Zustand (91) bis in den Ursprung (8) des Koordinatensystems in dem Koordinatensystem dargestellt wird.

5. Verfahren nach einem der Ansprüche 1 bis 4, **dadurch gekennzeichnet, dass** in dem Koordinatensystem verschiedene Gütebereiche (5, 6, 7) definiert und dargestellt werden, in denen der Zustand (91, 92, ..., 99) des Lebewesens unterschiedlich gut ist.

6. Verfahren nach einem der Ansprüche 1 bis 5, **dadurch gekennzeichnet, dass** der Zustand (91, 92, ..., 99) des Lebewesens in einem zweidimensionalen Koordinatensystem dargestellt wird.

7. Verfahren nach einem der Ansprüche 1 bis 6, **dadurch gekennzeichnet, dass** der physiologische Zustand (91, 92, ..., 99) des Lebewesens durch mindestens zwei der nachfolgend aufgeführten Größen Körpergewicht, Körperfettanteil, Körperwasseranteil, Muskelmassenanteil, Fluidbalance, die als das Verhältnis der Flüssigkeitsmenge außerhalb der Körperzellen zu der Flüssigkeitsmenge innerhalb der Körperzellen definiert ist, Bodymass-Index (BMI), der als das Verhältnis von Körpergewicht zu dem Quadrat der Körpergröße definiert ist, Blutdruck, Pulsfrequenz, Blutfett, Blutzucker oder Cholesterin des Lebewesens definiert wird.

8. Verfahren nach einem der Ansprüche 1 bis 6, **dadurch gekennzeichnet, dass** der physiologische Zustand (91, 92, ..., 99) des Lebewesens durch mindestens zwei der nachfolgend aufgeführten Größen Luftdruck, Umgebungstemperatur, Luftfeuchtigkeit, Lichtstärke, Lichtverteilung oder Schalllautstärke in der Umgebung definiert wird.

9. Verfahren nach einem der Ansprüche 1 bis 8, **dadurch gekennzeichnet, dass** die Geschwindigkeit der Änderung eines Zustands (91, 92, ..., 99) des Lebewesens durch eine auf die Zeitbasis bezogene Codierung der dargestellten Zustände (91, 92, ..., 99) in dem Koordinatensystem dargestellt wird.

10. Verfahren nach Anspruch 9, **dadurch gekennzeichnet, dass** die auf die Zeitbasis bezogene Codierung über unterschiedliche Strichstärken, unterschiedliche Farben oder unterschiedlichen Kontrast der dargestellten Zustände (91, 92, ..., 99) oder von Verbindungslinien zwischen den dargestellten Zuständen (91, 92, ..., 99) erzielt wird.

11. Verfahren nach einem der Ansprüche 1 bis 10, **dadurch gekennzeichnet, dass** die Werte jeder Größe in beliebigen zeitlichen Abständen vorliegen und bei mehreren Werten einer Größe pro Zeitbasis die Werte der Größe zu einem gemeinsamen Wert der Größe zusammengefaßt werden und der Zustand (91, 92, ..., 99) des Lebewesens auf Grundlage der Zeitbasis in Abhängigkeit von dem gemeinsamen Wert der Größe in dem Koordinatensystem dargestellt wird.

12. Verfahren nach einem der Ansprüche 1 bis 10, **dadurch gekennzeichnet, dass** die Werte jeder Größe in beliebigen zeitlichen Abständen vorliegen und bei weniger als einem Wert für eine Größe pro Zeitbasis fehlende Werte der Größe aus den vorhandenen Werten der Größe durch Ausgleichsrechnung bestimmt werden und der Zustand (91, 92, ..., 99) des Lebewesens auf Grundlage der Zeitbasis in Abhängigkeit von dem durch Ausgleichsrechnung bestimmten Wert der Größe in dem Koordinatensystem dargestellt wird.

13. Verfahren nach einem der Ansprüche 1 bis 8, **dadurch gekennzeichnet, dass** die Werte jeder Größe in der Zeitbasis entsprechenden äquidistanten zeitlichen Abständen vorliegen.

14. Verfahren zur Darstellung eines durch mehrere Größen definierten physiologischen Zustands eines Lebewesens insbesondere nach einem der Ansprüche 3 bis 13, wobei ein Ist-Verlauf des Zustands und ein idealer Verlauf des Zustands aufgenommen wird, **dadurch gekennzeichnet, dass** ein Erfolgsindex (11) ermittelt und dargestellt wird, der sich aus einem Vergleich des idealen Verlaufs (10) mit dem tatsächlichen Verlauf (9) des Zustands (91, 92, ..., 99) des Lebewesens ergibt.

15. Verfahren nach Anspruch 14, **dadurch gekennzeichnet, dass** für einen beliebigen Zustand (91) ein Vergleich eines Verbindungsvektors (12) zu einem nachfolgend erfassten Zustand (92) mit einem Verbindungsvektor (13) zu einem nachfolgenden Zustand (102) des idealen Verlaufs (10) ausgeführt wird.

16. Anzeigevorrichtung (1) zur Darstellung eines durch mehrere Größen definierten Zustands (91, 92, ..., 99) eines Lebewesens, wobei die Anzeigevorrichtung (1) einen Bildschirm (2) zur Darstellung des Zustands (91, 92, ..., 99) aufweist, **dadurch gekennzeichnet, dass** auf dem Bildschirm (2) ein mehrdimensionales kartesisches Koordinatensystem definiert ist, wobei jeweils einer Achse (3, 4) des Koordinatensystems eine der Größen zugeordnet ist, und auf dem Bildschirm (2) der Zustand (91, 92, ..., 99) des Lebewesens in dem Koordinatensystem darstellbar ist, wobei die Darstellung auf Grundlage einer vorgebbaren Zeitbasis in Abhängigkeit von den Werten der Größen erfolgt.

17. Steuergerät (40) einer Anzeigevorrichtung (1) zur Darstellung eines durch mehrere Größen definierten Zustands (91, 92, ..., 99) eines Lebewesens, **dadurch gekennzeichnet, dass** das Steuergerät (40) die Definition eines mehrdimensionalen kartesischen Koordinatensystems auf Mitteln (2) der Anzeigevorrichtung (1) veranlasst, wobei jeweils einer Achse (3, 4) des Koordinatensystems eine der Größen zugeordnet ist, und das Steuergerät (40) die Darstellung des Zustands (91, 92, ..., 99) des Lebewesens, in dem Koordinatensystem auf den Mitteln (2) veranlasst, wobei die Darstellung auf Grundlage einer vorgebbaren Zeitbasis in Abhängigkeit von den Werten der Größen erfolgt.

18. Speicherelement (46), insbesondere Random-Access-Memory, Read-Only-Memory oder Flash-Memory, für ein Steuergerät (40) einer Anzeigevorrichtung (1) zur Darstellung eines durch mehrere Größen definierten Zustands (91, 92, ..., 99) eines Lebewesens, wobei auf dem Speicherelement (46) ein Programm abgespeichert ist, das auf einem Rechengerät, insbesondere auf einem Mikroprozessor (47), ablauffähig und zur Ausführung eines Verfahrens nach einem der Ansprüche 1 bis 15 geeignet ist.

19. Computerprogramm, **dadurch gekennzeichnet, dass** es zur Durchführung des Verfahrens nach einem der Ansprüche 1 bis 15 geeignet ist, wenn es auf einem Rechengerät, insbesondere auf einem Mikroprozessor (47), ausgeführt wird.

20. Computerprogramm, **dadurch gekennzeichnet, dass** es auf einem Speicherelement (47), insbesondere auf einem Flash-Memory, abgespeichert ist.
